# EUROPEAN PATENT SPECIFICATION

(11) **EP 4 044 977 B1**
(45) Date of publication and mention of the grant of the patent: **27.03.2024**
(21) Application number: 20792328.5
(22) Date of filing: 09.10.2020
(51) Int. Cl.: A61F 13/00, A61B 5/00, A61F 13/02

(54) **SAFETY CIRCUITS FOR IMAGING WITH SENSOR INTEGRATED DRESSINGS AND SYSTEMS**
SICHERHEITSSCHALTUNGEN ZUR BILDGEBUNG MIT SENSORINTEGRIERTEN VERBÄNDEN UND SYSTEMEN
CIRCUITS DE SÉCURITÉ POUR IMAGERIE AVEC DES SYSTÈMES ET DES PANSEMENTS À CAPTEUR INTÉGRÉ

(30) Priority: 14.10.2019 GB 201914807
(43) Date of publication of application: 24.08.2022
(73) Proprietor: T.J.Smith and Nephew,Limited, Hull HU3 2BN (GB)
(72) Inventor: ASKEM, Ben, Alan, Hull, HU3 2BN (GB); HUNT, Allan, Kenneth, Frazer, Grugeon, Hull, HU3 2BN (GB); KELBIE, William, Inverness, IV3 8QW (GB)
(74) Representative: Smith & Nephew
(86) International application number: PCT/EP2020/078378
(87) International publication number: WO 2021/074022

(56) References cited:
- WO-A1-2019/193141
- WO-A1-2020/157103
- WO-A2-2019/063488

## Description

### Field

Embodiments of the present disclosure relate to apparatuses, systems, and methods for the monitoring and/or treatment of tissue with sensor integrated or sensor-enabled dressings.

### Description of the Related Art

Nearly all areas of medicine may benefit from improved information regarding the state of the tissue, organ, or system to be treated, particularly if such information is gathered in real-time during treatment, many types of treatments are still routinely performed without the use of sensor data collection. Instead, such treatments rely upon visual inspection by a caregiver or other limited means rather than quantitative sensor data. For example, in the case of wound treatment via dressings and/or negative pressure wound therapy, data collection is generally limited to visual inspection by a caregiver and often the underlying wounded tissue may be obscured by bandages or other visual impediments. Even intact, unwounded skin may have underlying damage that is not visible to the naked eye, such as a compromised vascular or deeper tissue damage that may lead to an ulcer or injury. Similar to wound treatment, during orthopedic treatments requiring the immobilization of a limb with a cast or other encasement, only limited information is gathered on the underlying tissue. In instances of internal tissue repair, such as a bone plate, continued direct sensor-driven data collection is not performed. Further, braces and/or sleeves used to support musculoskeletal function do not monitor the functions of the underlying muscles or the movement of the limbs. Outside of direct treatments, common hospital room items such as beds and blankets could be improved by adding capability to monitor patient parameters. Wound dressings comprising an optical sensor and a light source are disclosed in WO 2019/063488 A2, WO 2019/193141 A1 and WO 2020/157103 A1.

Therefore, there is a need for improved sensor monitoring, particularly through the use of sensor integrated substrates which can be incorporated into existing treatment regimes.

### SUMMARY

A wound dressing according to the invention is defined in the appended set of claims 1-16.

### BRIEF DESCRIPTION OF THE DRAWINGS

Embodiments of the present disclosure will now be described hereinafter, by way of example only, with reference to the accompanying drawings in which:
FIG. 1A illustrates a perspective view of a substrate supporting electronic components;
FIGS. 1B-1C illustrate perspective and top views of a perforated substrate supporting electronic components;
FIGS. 2A-2B illustrates cross-sections of wound dressings;
FIGS. 3A-3B illustrate perspective and top views of a perforated substrate supporting electronic components; and
FIGS. 4A-4D illustrate safety circuits.

### DETAILED DESCRIPTION

Embodiments disclosed herein relate to apparatuses and methods of at least one of monitoring or treating biological tissue with sensor-enabled substrates. The embodiments disclosed herein are not limited to treatment or monitoring of a particular type of tissue or injury, instead the sensor-enabled technologies disclosed herein are broadly applicable to any type of therapy that may benefit from sensor-enabled substrates. Some implementations utilize sensors and data collection relied upon by health care providers to make both diagnostic and patient management decisions.

Certain embodiments disclosed herein relate to the use of sensors mounted on or embedded within substrates configured to be used in the treatment of both intact and damaged human or animal tissue. Such sensors may collect information about the surrounding tissue and transmit such information to a computing device or a caregiver to be utilized in further treatment. In certain implementations, such sensors may be attached to the skin anywhere on the body, including areas for monitoring arthritis, temperature, or other areas that may be prone to problems and require monitoring. Sensors disclosed herein may also incorporate markers, such as radiopaque markers, to indicate the presence of the device, for example prior to performing an MRI or other technique.

The sensor embodiments disclosed herein may be used in combination with clothing. Non-limiting examples of clothing for use with embodiments of the sensors disclosed herein include shirts, pants, trousers, dresses, undergarments, outer-garments, gloves, shoes, hats, and other suitable garments. In certain embodiments, the sensor embodiments disclosed herein may be welded into or laminated into/onto the particular garments. The sensor embodiments may be printed directly onto the garment and/or embedded into the fabric. Breathable and printable materials such as microporous membranes may also be suitable.

Sensor embodiments disclosed herein may be incorporated into cushioning or bed padding, such as within a hospital bed, to monitor patient characteristics, such as any characteristic disclosed herein. In certain embodiments, a disposable film containing such sensors could be placed over the hospital bedding and removed/replaced as needed.

In some implementations, the sensor embodiments disclosed herein may incorporate energy harvesting, such that the sensor embodiments are self-sustaining. For example, energy may be harvested from thermal energy sources, kinetic energy sources, chemical gradients, or any suitable energy source.

The sensor embodiments disclosed herein may be utilized in rehabilitation devices and treatments, including sports medicine. For example, the sensor embodiments disclosed herein may be used in braces, sleeves, wraps, supports, and other suitable items. Similarly, the sensor embodiments disclosed herein may be incorporated into sporting equipment, such as helmets, sleeves, and/or pads. For example, such sensor embodiments may be incorporated into a protective helmet to monitor characteristics such as acceleration, which may be useful in concussion diagnosis.

The sensor embodiments disclosed herein may be used in coordination with surgical devices, for example, the NAVIO surgical system by Smith & Nephew Inc. In some implementations, the sensor embodiments disclosed herein may be in communication with such surgical devices to guide placement of the surgical devices. In some implementations, the sensor embodiments disclosed herein may monitor blood flow to or away from the potential surgical site or ensure that there is no blood flow to a surgical site. Further surgical data may be collected to aid in the prevention of scarring and monitor areas away from the impacted area.

To further aid in surgical techniques, the sensors disclosed herein may be incorporated into a surgical drape to provide information regarding tissue under the drape that may not be immediately visible to the naked eye. For example, a sensor embedded flexible drape may have sensors positioned advantageously to provide improved area-focused data collection. In certain implementations, the sensor embodiments disclosed herein may be incorporated into the border or interior of a drape to create fencing to limit/ control the surgical theater.

Sensor embodiments as disclosed herein may also be utilized for pre-surgical assessment. For example, such sensor embodiments may be used to collect information about a potential surgical site, such as by monitoring skin and the underlying tissues for a possible incision site. For example, perfusion levels or other suitable characteristics may be monitored at the surface of the skin and deeper in the tissue to assess whether an individual patient may be at risk for surgical complications. Sensor embodiments such as those disclosed herein may be used to evaluate the presence of microbial infection and provide an indication for the use of antimicrobials. Further, sensor embodiments disclosed herein may collect further information in deeper tissue, such as identifying pressure ulcer or pressure injury damage and/or the fatty tissue levels.

The sensor embodiments disclosed herein may be utilized in cardiovascular monitoring. For example, such sensor embodiments may be incorporated into a flexible cardiovascular monitor that may be placed against the skin to monitor characteristics of the cardiovascular system and communicate such information to another device and/or a caregiver. For example, such a device may monitor pulse rate, oxygenation of the blood, and/or electrical activity of the heart. Similarly, the sensor embodiments disclosed herein may be utilized for neurophysiological applications, such as monitoring electrical activity of neurons.

The sensor embodiments disclosed herein may be incorporated into implantable devices, such as implantable orthopedic implants, including flexible implants. Such sensor embodiments may be configured to collect information regarding the implant site and transmit this information to an external source. In some cases, an internal source may also provide power for such an implant.

The sensor embodiments disclosed herein may also be utilized for monitoring biochemical activity on the surface of the skin or below the surface of the skin, such as lactose buildup in muscle or sweat production on the surface of the skin. In some cases, other characteristics may be monitored, such as glucose concentration, urine concentration, tissue pressure, skin temperature, skin surface conductivity, skin surface resistivity, skin hydration, skin maceration, and/or skin ripping.

Sensor embodiments as disclosed herein may be incorporated into Ear, Nose, and Throat (ENT) applications. For example, such sensor embodiments may be utilized to monitor recovery from ENT-related surgery, such as wound monitoring within the sinus passage.

Sensor embodiments disclosed herein may encompass sensor printing technology with encapsulation, such as encapsulation with a polymer film. Such a film may be constructed using any polymer described herein, such as polyurethane. Encapsulation of the sensor embodiments may provide waterproofing of the electronics and protection from local tissue, local fluids, and other sources of potential damage.

In certain embodiments, the sensors disclosed herein may be incorporated into an organ protection layer. Such a sensor-embedded organ protection layer may both protect the organ of interest and confirm that the organ protection layer is in position and providing protection. Further, a sensor-embedded organ protection layer may be utilized to monitor the underlying organ, such as by monitoring blood flow, oxygenation, and other suitable markers of organ health. In some cases, a sensor-enabled organ protection layer may be used to monitor a transplanted organ, such as by monitoring the fat and muscle content of the organ. Further, sensor-enabled organ protection layers may be used to monitor an organ during and after transplant, such as during rehabilitation of the organ.

The sensor embodiments disclosed herein may be incorporated into treatments for wounds (disclosed in greater detail below) or in a variety of other applications. Non-limiting examples of additional applications for the sensor embodiments disclosed herein include: monitoring and treatment of intact skin, cardiovascular applications such as monitoring blood flow, orthopedic applications such as monitoring limb movement and bone repair, neurophysiological applications such as monitoring electrical impulses, and any other tissue, organ, system, or condition that may benefit from improved sensor-enabled monitoring.

### Wound Therapy

Some systems and methods disclosed herein relate to wound therapy for a human or animal body. Therefore, any reference to a wound herein can refer to a wound on a human or animal body, and any reference to a body herein can refer to a human or animal body. The disclosed technology embodiments may relate to preventing or minimizing damage to physiological tissue or living tissue, or to the treatment of damaged tissue (for example, a wound as described herein) wound with or without reduced pressure, including for example a source of negative pressure and wound dressing components and apparatuses. The apparatuses and components comprising the wound overlay and packing materials or internal layers, if any, are sometimes collectively referred to herein as dressings. In some cases, the wound dressing can be provided to be utilized without reduced pressure.

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno-compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, or epidermolysis bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, pressure injury, stoma, surgical wounds, trauma and venous ulcers or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

Wounds may also include a pressure injury. A pressure injury is localized damage to the skin and/or underlying soft tissue, usually over a bony prominence or related to a medical or other device. The injury can present as intact skin or an open ulcer and may be painful. The injury occurs as a result of intense and/or prolonged pressure or pressure in combination with shear. The tolerance of soft tissue for pressure and shear may also be affected by microclimate, nutrition, perfusion, comorbidities and condition of the soft tissue.

Wound may also include tissue at risk of becoming a wound as discussed herein. For example, tissue at risk may include tissue over a bony protuberance (at risk of deep tissue injury/insult) or pre-surgical tissue (for example, knee tissue) that may have the potential to be cut (for example, for joint replacement/surgical alteration/reconstruction).

Some systems and methods disclosed herein relate to methods of treating a wound with the technology disclosed herein in conjunction with one or more of the following: advanced footwear, turning a patient, offloading (such as, offloading diabetic foot ulcers), treatment of infection, systemix, antimicrobial, antibiotics, surgery, removal of tissue, affecting blood flow, physiotherapy, exercise, bathing, nutrition, hydration, nerve stimulation, ultrasound, electrostimulation, oxygen therapy, microwave therapy, active agents ozone, antibiotics, antimicrobials, or the like.

Alternatively or additionally, a wound may be treated using topical negative pressure (TNP) and/or traditional advanced wound care, which is not aided by the using of applied negative pressure (may also be referred to as non-negative pressure therapy).

Advanced wound care may include use of an absorbent dressing, an occlusive dressing, use of an antimicrobial and/or debriding agents in a wound dressing or adjunct, a pad (for example, a cushioning or compressive therapy, such as stockings or bandages), or the like.

In some cases, a wound dressing comprises one or more absorbent layer(s). The absorbent layer may be a foam or a superabsorbent.

In some cases, the disclosed technology may be used in conjunction with a non-negative pressure dressing. A non-negative pressure wound dressing suitable for providing protection at a wound site may comprise an absorbent layer for absorbing wound exudate and an obscuring element for at least partially obscuring a view of wound exudate absorbed by the absorbent layer in use. The obscuring element may be partially translucent. The obscuring element may be a masking layer.

In some cases, the non-negative pressure wound dressing as disclosed herein comprises the wound contact layer and the absorbent layer overlies the wound contact layer. The wound contact layer can carry an adhesive portion for forming a substantially fluid tight seal over the wound.

In some cases, the wound dressing as disclosed herein further comprises layer of a superabsorbent fiber, or a viscose fiber or a polyester fiber.

In some cases, the wound dressing as disclosed herein further comprises a backing layer. The backing layer may be a transparent or opaque film. Typically the backing layer comprises a polyurethane film (typically a transparent polyurethane film).

In some cases, the foam may be an open cell foam, or closed cell foam, typically an open cell foam. The foam can be hydrophilic.

The wound dressing may comprise a transmission layer and the layer can be foam. The transmission layer may be a polyurethane foam laminated to a polyurethane film.

The non-negative pressure wound dressing may be a compression bandage. Compression bandages are known for use in the treatment of oedema and other venous and lymphatic disorders, e.g., of the lower limbs. The compression bandage in some cases may comprise a bandage system comprising an inner skin facing layer and an elastic outer layer, the inner layer comprising a first ply of foam and a second ply of an absorbent nonwoven web, the inner layer and outer layer being sufficiently elongated so as to be capable of being wound about a patient's limb.

### Negative Pressure Wound Therapy

In some cases, treatment of wounds can be performed using negative pressure wound therapy. It will be understood that embodiments of the present disclosure are generally applicable to use in TNP systems. Briefly, negative pressure wound therapy assists in the closure and healing of many forms of "hard to heal" wounds by reducing tissue oedema; encouraging blood flow and granular tissue formation; removing excess exudate and may reduce bacterial load (and thus infection risk). In addition, the therapy allows for less disturbance of a wound leading to more rapid healing. TNP therapy systems may also assist on the healing of surgically closed wounds by removing fluid and by helping to stabilize the tissue in the apposed position of closure. A further beneficial use of TNP therapy can be found in grafts and flaps where removal of excess fluid is important and close proximity of the graft to tissue is required in order to ensure tissue viability.

Negative pressure therapy can be used for the treatment of open or chronic wounds that are too large to spontaneously close or otherwise fail to heal by means of applying negative pressure to the site of the wound. Topical negative pressure (TNP) therapy or negative pressure wound therapy (NPWT) involves placing a cover that is impermeable or semi-permeable to fluids over the wound, using various means to seal the cover to the tissue of the patient surrounding the wound, and connecting a source of negative pressure (such as a vacuum pump) to the cover in a manner so that negative pressure is created and maintained under the cover. In some cases, the source of negative pressure can be supported by a wound dressing positioned in and/or over the wound. It is believed that such negative pressures promote wound healing by facilitating the formation of granulation tissue at the wound site and assisting the body's normal inflammatory process while simultaneously removing excess fluid, which may contain adverse cytokines or bacteria.

Some of the dressings used in NPWT can include many different types of materials and layers, for example, gauze, pads, foam pads or multi-layer wound dressings. One example of a multi-layer wound dressing is the PICO dressing, available from Smith & Nephew, includes a wound contact layer and a superabsorbent layer beneath a backing layer to provide a canister-less system for treating a wound with NPWT. The wound dressing may be sealed to a suction port providing connection to a length of tubing, which may be used to pump fluid out of the dressing or to transmit negative pressure from a pump to the wound dressing. Additionally, RENASYS-F, RENASYS-G, RENASYS-AB, and RENASYS-F/AB, available from Smith & Nephew, are additional examples of NPWT wound dressings and systems. Another example of a multi-layer wound dressing is the ALLEVYN Life dressing, available from Smith & Nephew, which includes a moist wound environment dressing that is used to treat the wound without the use of negative pressure.

As is used herein, reduced or negative pressure levels, such as -X mmHg, represent pressure levels relative to normal ambient atmospheric pressure, which can correspond to 760 mmHg (or 1 atm, 29.93 inHg, 101.325 kPa, 14.696 psi, etc.). Accordingly, a negative pressure value of -X mmHg reflects absolute pressure that is X mmHg below 760 mmHg or, in other words, an absolute pressure of (760-X) mmHg. In addition, negative pressure that is "less" or "smaller" than X mmHg corresponds to pressure that is closer to atmospheric pressure (such as, -40 mmHg is less than -60 mmHg). Negative pressure that is "more" or "greater" than -X mmHg corresponds to pressure that is further from atmospheric pressure (such as, - 80 mmHg is more than -60 mmHg). In some cases, local ambient atmospheric pressure is used as a reference point, and such local atmospheric pressure may not necessarily be, for example, 760 mmHg.

In some cases of wound closure devices described herein, increased wound contraction can lead to increased tissue expansion in the surrounding wound tissue. This effect may be increased by varying the force applied to the tissue, for example by varying the negative pressure applied to the wound over time, possibly in conjunction with increased tensile forces applied to the wound via embodiments of the wound closure devices. In some cases, negative pressure may be varied over time for example using a sinusoidal wave, square wave, or in synchronization with one or more physiological indices (such as, heartbeat).

Any of the embodiments disclosed herein can be used in combination with any of the features disclosed in one or more of WO2010/061225, US2016/114074, US2006/0142560, and US5,703,225, which describe absorbent materials; WO2013/007973, which describes non-negative pressure wound dressings; GB1618298.2 (filed on 28 October 2016), GB1621057.7 (filed on 12 December 2016), and GB1709987.0 (filed on 22 June 2017), which describe multi-layered wound dressings; EP2498829 and EP1718257, which describe wound dressings; WO2006/110527, US 6,759,566, and US2002/0099318, which describe compression bandages; US8,235,955 and US7,753,894, which describe wound closure devices; WO2013/175306, WO2016/174048, US2015/0190286, US2011/0282309, and US2016/0339158, which describe negative pressure wound therapy dressings, wound dressing components, wound treatment apparatuses, and methods.

### Substrate Supporting Sensors

A wound dressing that incorporates a number of electronic components, including one or more sensors, can be utilized in order to monitor characteristics of a wound. Collecting and analyzing data from a wound can provide useful insights towards determining whether a wound is on a healing trajectory, selecting proper therapy, determining whether the wound has healed, or the like.

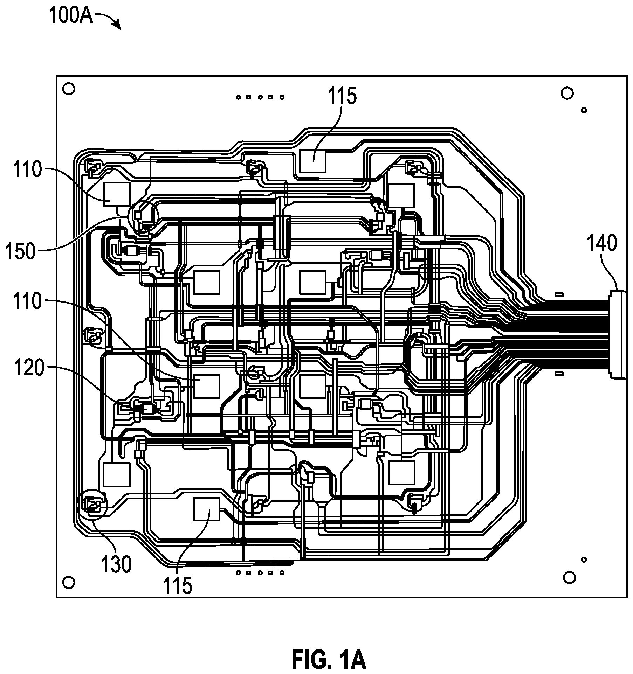

In some implementations, a number of sensor technologies can be used in wound dressings or one or more components forming part of an overall wound dressing apparatus. For example, as illustrated in FIGS. 1A-1C, one or more sensors can be incorporated onto or into a substrate (such substrate can be referred to as "sensor integrated substrate"). The substrate illustrated as having a square shape, but it will be appreciated that the substrate may have other shapes such as rectangular, circular, oval, etc. In some cases, a substrate supporting one or more sensors can be provided as an individual material layer that is placed directly or indirectly over or in a wound. The sensor integrated substrate can be part of a larger wound dressing apparatus. In some cases, the sensor integrated substrate is part of a single unit dressing. Additionally or alternatively, the sensor integrated substrate can be placed directly or indirectly over or in the wound and then covered by a secondary wound dressing, which can include one or more of gauze, foam or other wound packing material, a superabsorbent layer, a drape, a fully integrated dressing like the Pico or Allevyn Life dressing manufactured by Smith & Nephew, or the like.

The sensor integrated substrate can be placed in contact with a wound and can allow fluid to pass through the substrate while causing little to no damage to the tissue in the wound. The substrate can be flexible, elastic, extensible, or stretchable or substantially flexible, elastic, extensible, or stretchable in order to conform to or cover the wound. For example, the substrate can be made from a stretchable or substantially stretchable material, such as one or more of polyurethane, thermoplastic polyurethane (TPU), silicone, polycarbonate, polyethylene, polyimide, polyamide, polyester, polyethelene tetraphthalate (PET), polybutalene tetreaphthalate (PBT), polyethylene naphthalate (PEN), polyetherimide (PEI), along with various fluropolymers (FEP) and copolymers, or another suitable material.

In some cases, the substrate can include one or more flexible circuit boards, which can be formed of flexible polymers, including polyamide, polyimide (PI), polyester, polyethylene naphthalate (PEN), polyetherimide (PEI), along with various fluropolymers (FEP) and copolymers, or the like. One or more sensors can be incorporated into a two-layer flexible circuit board. In some scenarios, the one or more circuit boards can be a multi-layer flexible circuit board.

In some cases, the sensor integrated substrate can incorporate adhesive, such as a wound contact layer as described herein, that adheres to wet or dry tissue. In some cases, one or more sensors, which can be positioned one or more flexible circuit boards, can be incorporated into any layer of the wound dressing. For example, a wound contact layer can have cutouts or slits that allow for one or more sensors to protrude out of the lower surface of the wound contact layer and contact the wound directly. In some situations, one or more sensors can be incorporated into or encapsulated within other components of a wound dressing, such as an absorbent layer.

As shown in FIG. 1A, a sensor integrated substrate 100A can support a plurality of electronic components and a plurality of electronic connections interconnecting at least some of the components. The electronic components can be one or more of any electronic components described herein, such as a sensor, amplifier, capacitor, resistor, inductor, controller, processor, or the like. The electronic connections can electrically connect one or more of the electronic components. The electronic connections can be can be tracks printed on the substrate, such as using copper, conductive ink (such as silver ink, graphite ink, etc.), or the like. At least some of the electronic connections can be flexible or stretchable or substantially flexible or stretchable.

The plurality of electronic components can include one or more impedance or conductivity sensors 110, which can be arranged in an outer 4x4 grid and an inner 4x4 grid as illustrated in FIGS. 1A-1C. Sensors 110 are illustrated as pads configured to measure impedance or conductivity of tissue across any pair of the pads. Two (or more) excitation pads 115 can be arranged as illustrated to provide the excitation signal across the pads, which is conducted by the tissue and responsive to which impedance or conductance of the tissue can be measured across the pads 110. Electrical components, such as one or more amplifiers 120, can be used to measure impedance or conductance of the tissue. Impedance or conductance measurements can be used to identify living and dead tissue, monitor progress of healing, or the like. The arrangement of the pads 110 in the inner and outer grids can be used to measure the impedance or conductance of the wound, perimeter of the wound, or tissue or areas surrounding the wound.

The plurality of electronic components can include one or more temperature sensors 130 configured to measure temperature of the wound or surrounding tissue. For example, nine temperature sensors arranged around the perimeter of the substrate 100A. One or more temperature sensors can include one or more thermocouples or thermistors. One or more temperature sensors can be calibrated and the data obtained from the one or more sensors can be processed to provide information about the wound environment. In some cases, an ambient sensor measuring ambient air temperature can also be used to assist in eliminating problems associated with environment temperature shifts.

The plurality of electronic components can include one or more optical sensors 150. One or more optical sensors 150 can be configured to measure wound appearance or image the wound. In some cases, a light source or illumination source that emits light and a light sensor or detector that detects light reflected by the wound are used as one or more optical sensors. The light source can be a light emitting diode (LED), such as one or more of white LED, red, green, blue (RGB) LED, ultraviolet (UV) LED, or the like. The light sensor can be one or more of an RGB sensor configured to detect color, infrared (IR) color sensor, UV sensor, or the like. In some cases, both the light source and detector would be pressed up against the skin, such that light would penetrate into the tissue and take on the spectral features of the tissue itself. In some scenarios, one or more optical sensor can include an imaging device, such as a charge-coupled device (CCD), CMOS image sensor, or the like.

In some cases, ultra bright LEDs, an RGB sensor, and polyester optical filters can be used as components of the one or more optical sensors to measure through tissue color differentiation. For example, because surface color can be measured from reflected light, a color can be measured from light which has passed through the tissue first for a given geometry. This can include color sensing from diffuse scattered light, from an LED in contact with the skin, or the like. In some cases, an LED can be used with a proximal RGB sensor to detect the light which has diffused through the tissue. The optical sensors can image with diffuse internal light or surface reflected light.

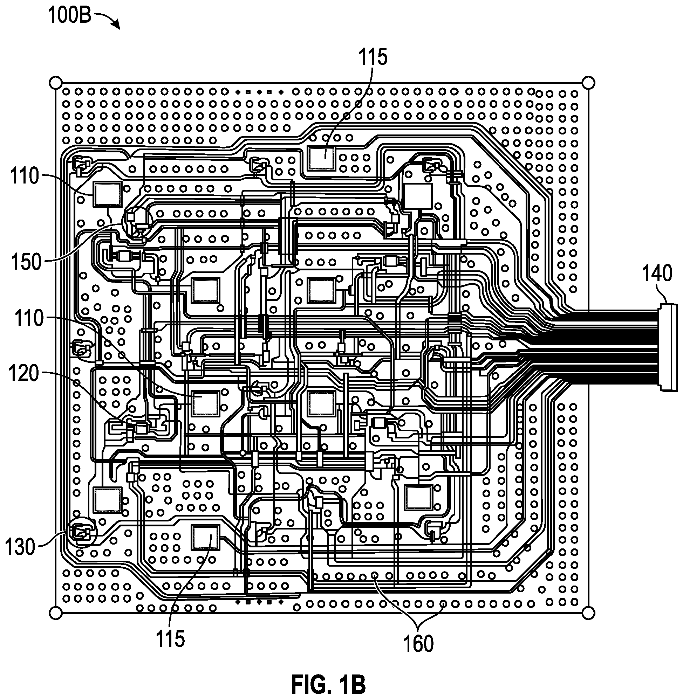

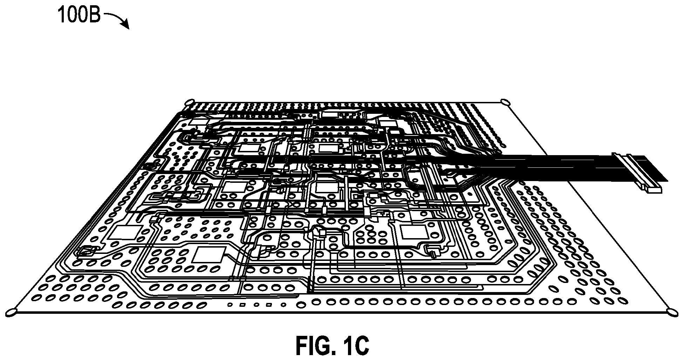

One or more of the plurality of electronic components can be controlled by a control module. The control module can receive and process one or more measurements obtained by the one or more sensors. An external control module can be connected to at least some of the plurality of electronic components via a connector 140. In some cases, the connector 140 can be positioned at the end of a conductive track portion as illustrated in FIG. 1B or attached to the conductive track portion at a position away from the end as illustrated in FIG. 1A or 1C (such as, attached to the top of the track portion with glue). The control module can include one or more controllers or microprocessors, memory, or the like. In some cases, one or more controllers can be positioned on the substrate, and the connector 140 is not used. In some cases, data and commands can be communicated wirelessly, such as by a transceiver positioned on the substrate, and the connector 140 is not used.

In some cases, additional or alternative sensors can be positioned on the substrate, such as one or more pH sensors, pressure sensors, perfusion sensors, or the like.

In some cases, a substrate can be perforated as illustrated in FIGS. 1 B-1C. A plurality of perforations 160 can be formed in the substrate 100B, allowing fluid to pass through the substrate. It may be advantageous to use a perforated substrate in conjunction with application of negative pressure wound therapy, during which reduced pressure is applied to the wound covered by a dressing and which causes removal of fluid (such as wound exudate) from the wound. Perforations 160 can be formed around a plurality of electronic components and connections as illustrated in FIGS. 1 B-1C. Perforations 160 can be formed as slits or holes. In some cases, perforations 160 can be small enough to help prevent tissue ingrowth while allowing fluid to pass through the substrate.

In some cases, any of the wound dressings or wound dressing components described herein can be part of a kit that also includes a negative pressure wound therapy device. One or more components of the kit, such as the sensor integrated substrate, secondary dressing, or the negative pressure wound therapy device can be sterile. Any of the embodiments disclosed herein can be used with any of the embodiments described in International Patent Publication No. WO2017/195038, titled "SENSOR ENABLED WOUND MONITORING AND THERAPY APPARATUS," International Patent Publication No. WO2018/189265, titled "COMPONENT STRESS RELIEF FOR SENSOR ENABLED NEGATIVE PRESSURE WOUND THERAPY DRESSINGS," International Patent Application No. PCT/EP2018/069886, titled "SKEWING PADS FOR IMPEDANCE MEASUREMENT," and International Patent Application No. PCT/EP2018/075815, titled "SENSOR POSITIONING AND OPTICAL SENSING FOR SENSOR ENABLED WOUND THERAPY DRESSINGS AND SYSTEMS,".

### Encapsulation and Stress Relief

In some cases, while it may be desirable for a substrate to be stretchable or substantially stretchable to better conform to or cover the wound, at least some of the electronic components or connections may not be stretchable or flexible. In such instances, undesirable or excessive localized strain or stress may be exerted on the one or more electronic components, such as on the supporting area or mountings of an electronic component, when the substrate is positioned in or over the wound. For example, such stress can be due to patient movement, changes in the shape or size of the wound (such as, due to its healing), or the like. Such stress may cause movement, dislodgment, or malfunction of the one or more electronic components or connections (for example, creation of an open circuit from a pin or another connector becoming disconnected). Alternatively or additionally, it may be desirable to maintain the position of one or more electronic components, such as one or more sensors, in the same or substantially same location or region with respect to the wound (such as, in contact with the wound) so that measurements collected by the one or more electronic components accurately capture changes over time in the same or substantially same location or region of the wound. While the surface of the stretchable substrate may move when, for example, the patient moves, it may be desirable to maintain same or substantially same locations of one or more electronic components relative to the wound.

To address these problems, in some cases, non-stretchable or substantially non-stretchable coating (such coating can sometimes be referred to as "hard coat") can be applied to one or more electronic components, one or more electronic connections, or the like. Hard coat can provide one or more of reinforcement or stress relief for one or more electronic components, one or more electronic connections, or the like. Hard coating can be formed from acrylated or modified urethane material. For example, hard coat can be one or more of Dymax 1901-M, Dymax 9001-E, Dymax 20351, Dymax 20558, Henkel Loctite 3211, or another suitable material. Hard coat can have viscosity from about 13,500cP to 50,000cP before being cured or from about 3,600cP to about 6,600cP before being cured. In some cases, hard coat can have viscosity of no more than about 50,000cP. Hard coat can have hardness from about D40 to about D65 and/or linear shrinkage of about 1.5-2.5%.

In some cases, another coating (or coatings) can be applied to encapsulate or coat one or more of the substrate or components supported by the substrate, such as the electronic connections or the electronic components. Coating can provide biocompatibility, shield or protect the electronics from coming into contact with fluids, provide padding for the electronic components to increase patient comfort, or the like. As used herein, biocompatible can mean being in compliance with one or more applicable standards, such as ISO 10993 or USP Class VI. Such coating cam be sometimes referred to as "conformal coat" or "soft coat." Soft coat can be stretchable or substantially stretchable. Soft coat can be hydrophobic or substantially hydrophobic.

Soft coat can be formed from one or more suitable polymers, adhesives, such as 1072-M adhesive (for example, Dymax 1072-M), 1165-M adhesive (such as, Dymax 1165-M), parylene (such as, Parylene C), silicones, epoxies, urethanes, acrylated urethanes, acrylated urethane alternatives (such as, Henkel Loctite 3381), or other suitable biocompatible and substantially stretchable materials. Soft coat can be thin coating, for example, from about 80 microns or less up to several millimeters or more. Soft coat can have hardness lower than about A100, A80, A50 or lower. Soft coat can have elongation at break higher than about 100%, 200%, 300% or more. Soft coat can have viscosity of about 8,000-14,500 centipoise (cP). In some cases, coating can have viscosity no less than about 3,000cP. In some cases, coating can have viscosity less than about 3,000cP.

Any of the hard or soft coats described herein can be applied by one or more of laminating, adhering, welding (for instance, ultrasonic welding), curing by one or more of light, UV, thermal (such as, heat), or the like. Any of the hard or soft coat described herein can be transparent or substantially transparent to facilitate optical sensing. Any of the coatings described herein can retain bond strength when subjected to sterilization, such as EtO sterilization. Any of the coatings described herein can be modified to fluoresce, such as under UV light.

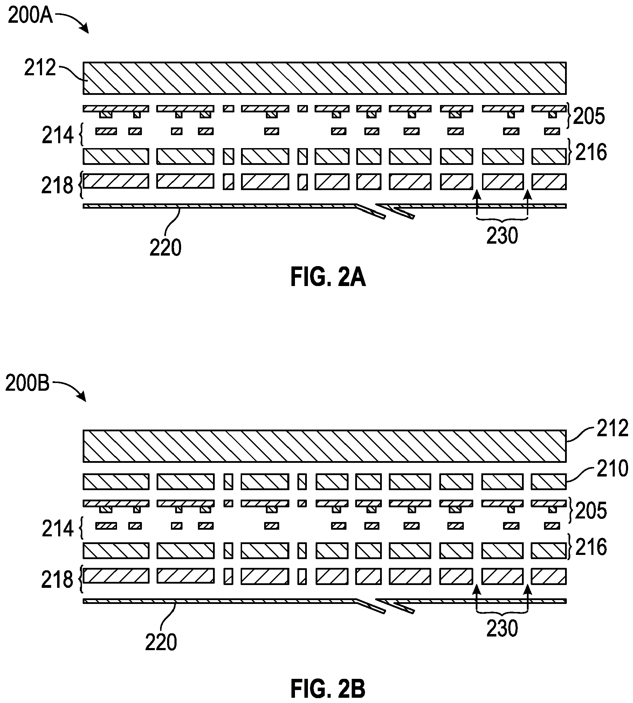

FIGS. 2A-2B illustrate cross-sections of wound dressings that include sensor integrated substrates. Dressing 200A shown in FIG. 2A can include a sensor integrated substrate 205 supporting a plurality of electronic components (shown as protruding from the substrate) and a plurality of electronic connections, as described herein. The dressing 200A can include hard coat 214, applied to one or more electronic components or connections. In some cases, hard coat can be applied to areas where electronic components are connected to electronic connections. This can reinforce these connections. In some cases, hard coat can be applied to each of the one or more of the electronic components or connections.

The dressing 200A can include soft coat 216, which can be applied to the entire wound facing side of the substrate. Soft coat 216 can be applied to an entire or substantially entire area of the wound facing side of the substrate to encapsulate the substrate, electronic components, and connections. In some cases, soft coat 216 can be applied to certain regions of the substrate, such as those regions supporting one or more of electronic components or connections.

The dressing 200A can include a wound contact layer 218. The wound contact layer 218 can include adhesive material configured to adhere the substrate to the wound, which can facilitate maintaining contact of one or more sensors with the wound. The wound contact layer 218 can be formed from silicone. The silicone material can be low tac (or tack) silicone. The wound contact layer 218 can include silicone adhesive mounted on a film. In some cases, the wound contact layer 218 can be similar to the material used in Allevyn Life Non-Bordered dressing manufactured by Smith & Nephew.

The wound contact layer 218 can be applied to entire or substantially entire area of the wound facing side of the substrate. In some cases, the wound contact layer 218 can be applied to certain regions of the substrate, such as those regions supporting one or more of electronic components or connections.

As illustrated in FIG. 2A, a plurality of perforations 230 can be formed through one or more of the substrate, hard coat, soft coat, and wound contact layer. As described herein, perforations can be made in regions or areas of the substrate that do not support electronic components or connections.

The dressing 200A can include a protective layer 220 applied to the wound contact layer 218. The protective layer 220 can be made of paper, such as laminated paper. The protective layer 220 can protect the wound contact layer 218 prior to use and facilitate easy application for a user. The protective layer 220 can include a plurality (such as two) handles. The handles can be applied in a folded configuration, in which a slit separating the handles is covered by one of handles folded over the slit. In some cases, the protective layer 220 can be similar to the protective layer used in the Allevyn Life Non-Bordered dressing.

As illustrated, a wicking layer 212 can be positioned over an opposite, non wound facing side of the substrate. The wicking layer 212 can facilitate passage of fluid through the layers below the wicking layer. For example, the wicking layer can transport (or "wick") fluid away from the lower layers, such as from the substrate, toward one or more upper layers positioned over the wicking layer 212. Such one or more upper layers can include one or more absorbent materials as described herein. In some cases, the wicking layer 212 is formed from foam, such as foam similar to that used in the Allevyn Life Non-Bordered dressing. The wicking layer can be extensible or substantially extensible.

As illustrated in the dressing 200B of FIG. 2B, additional layer of soft coat 210 can be positioned over the non-wound facing side of the substrate between the substrate and the wicking layer 212. For example, soft coat 210 can protect the non-wound facing side of the substrate from fluid if the substrate is formed from material that is not impermeable to fluid. In such case, soft coat 210 can be hydrophobic or substantially hydrophobic. Soft coat 210 can be made of same or different material than soft coat 218. Soft coat 210 can be perforated as illustrated and described. In some cases, soft coat can encapsulate the entire substrate, including both the wound facing and non-wound facing sides.

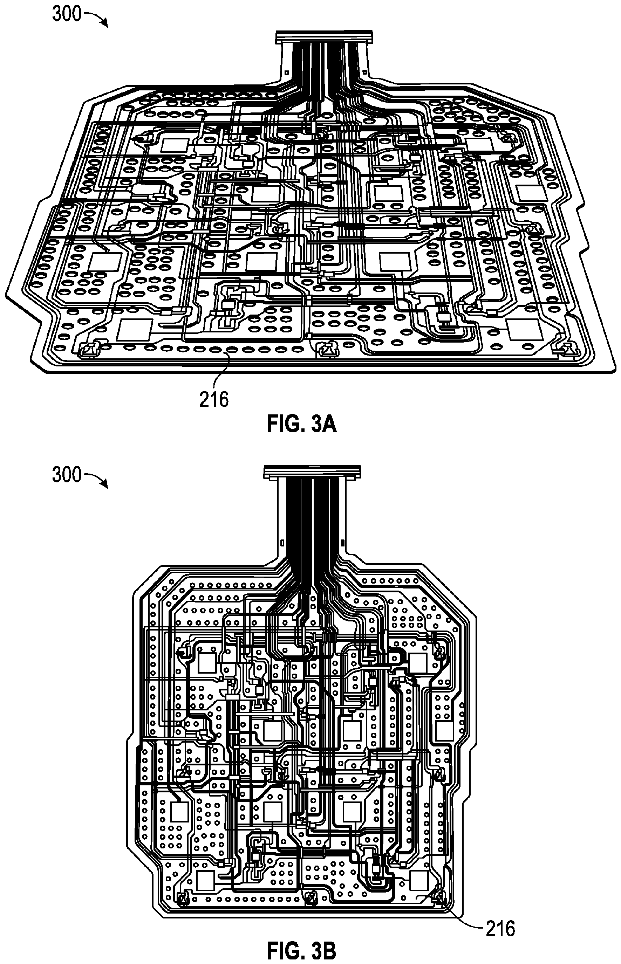

FIGS. 3A-3B illustrate coated sensor integrated substrates 300. The substrates 300 are illustrated with non-wound facing side 216 up. The substrates 300 can be similar to any of the substrates described herein.

Any of the embodiments disclosed herein can be used with any of the embodiments described in International Patent Application No.
PCT/EP2018/069883, titled "BIOCOMPATIBLE ENCAPSULATION AND COMPONENT STRESS RELIEF FOR SENSOR ENABLED NEGATIVE PRESSURE WOUND THERAPY DRESSINGS,".

### Safety Circuits

As described herein, the sensor integrated substrate can support a plurality of electronic components, including one or more optical sensors (for example, one or more optical sensors 150). An optical sensor can include a light source or illumination source (such as, an LED). In case of malfunction of at least some of electronic components and/or software or firmware controlling such components, there is a risk that the light source can become hot if it remains in active (or "on") state for a prolonged time period. This can cause discomfort or harm to the patient. Additionally or alternatively, this can violate one or more applicable safety protocols, such as International Electronics Commission (IEC) 60601-1 (in particular, the requirement for maximum temperature of applied parts).

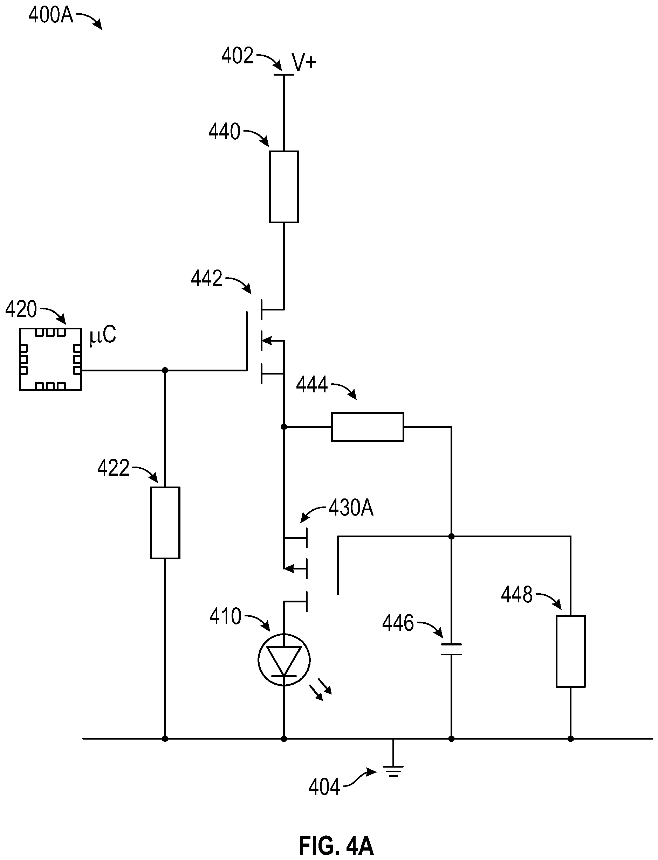

FIGS. 4A-4C illustrate safety circuits that solve the above problems. The illustrated circuits can deactivate the light source or limit power provided to the light source in order to prevent overheating.

FIG. 4A illustrates a safety circuit 400A. A switch 442 can control supply of power from a power supply 402. The switch 442 can be, for example, an n-type metal-oxide-semiconductor field-effect transistor (nMOSFET or nMOSFET transistor). In some cases, the switch 422 can be a p-type metal-oxide- semiconductor field-effect transistor (pMOSFET or pMOSFET transistor). A resistor 440 can be positioned between the switch 442 and the power supply 402. The resistor 440 can be a pull-down resistor, which can prevent any capacitively coupled signals from inadvertently activating the nMOSFET transistor. The switch 442 can be controlled by a controller 420. For example, the controller 420 can be connected to a gate of the nMOSFET transistor 442 and create an electric field at the gate of the transistor. The controller 420 can be connected to the gate of the nMOSFET transistor 442 via a resistor 422 connected to the controller and ground
404. Creation of electric field at the gate of the nMOSFET transistor 442 can cause the nMOSFET transistor to turn on (or cause the transistor to be in the active state). Ceasing creation of the electric field can cause the nMOSFET transistor 442 to turn off (or cause the transistor to be in the cutoff state). In the on state, the switch 442 can conduct electrical current. In the off state, the switch 442 can act as an open circuit and not conduct electrical current.

A switch 430A can control supply of power to a light source 410 (such as, an LED). The switch 430A can be, for example, a pMOSFET transistor (or an nMOSFET transistor in some cases). The state of the switch 430A (such as, on or off state) can be controlled by a circuit that includes one or more of a resistor 444, capacitor 446, or resistor 448. As illustrated, such circuit can be a resistor-capacitor (RC) circuit or network. The RC network can charge up a gate of the pMOSFET transistor 430A (to which the capacitor 446 can be connected), which can cause the transistor to transition from an on (or active) state in which the transistor 430A conducts to an off (or inactive or cutoff) state in which the transistor 430A does not conduct. Such transition can cause the pMOSFET transistor 430A to switch off, thereby preventing power (such as, flow of electrical current) from being supplied to the light source 410.

Circuit 400A can operate as follows. The controller 420 can create an electric field at the gate of the nMOSFET transistor 442 causing the transistor to turn on. This can cause the pMOSFET transistor 430A turn on resulting in power (such as, current or voltage) from the power source 402 being supplied to the light source 410. Power can also be supplied to the RC network. As power is being supplied to the RC network, capacitor 446 may be charged. Once the capacitor 446 becomes sufficiently charged, electrical energy (such as, voltage) supplied to the gate of the pMOSFET transistor 430A from the capacitor 446 can cause the pMOSFET transistor 430A to turn off. This can prevent power from being supplied to the light source 410, which can cause the light source 410 to turn off (or be deactivated). For example, the capacitor 446 can be charged to about 63.2% of applied direct current (DC) voltage (which can correspond to a time constant of the RC network). Capacitance of the capacitor 446 as well resistances of one or more resistors 444 and 448 can be selected to such that the time constant of the RC network corresponds to a time period associated with safe operation of the light source 410. For example, such time period can be associated with duration of time over which temperature of the light source 410 when the light source operates would not reach or satisfy an unsafe level. Additionally or alternatively, capacitance of the capacitor 446 can be selected to ensure that the capacitor can store sufficient charge to cause the pMOSFET transistor 430A to turn off.

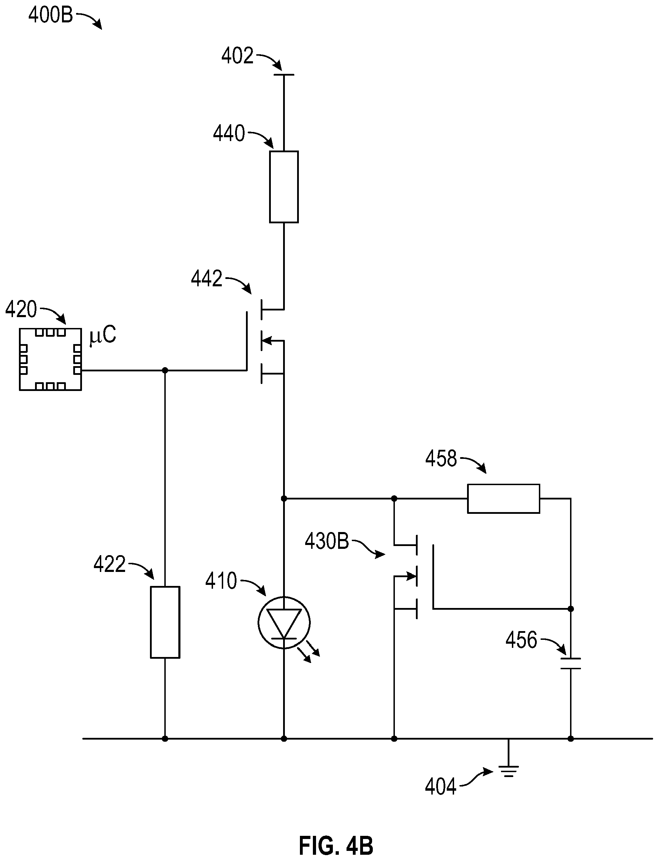

FIG. 4B illustrates a safety circuit 400B. The safety circuit 400B utilizes, as a switch 430B, an nMOSFET transistor in place of the pMOSFET transistor 430A in the circuit 400A. The state of the switch 430B (such as, on or off state) can be controlled by a circuit that includes one or more of a capacitor 456 or resistor 458. As illustrated, such circuit can be a resistor-capacitor (RC) circuit or network. The RC network can charge up a gate of the nMOSFET transistor 430B (to which the capacitor 456 can be connected), which can cause the transistor to transition from an off (or inactive or cutoff) state in which the transistor 430B does not conduct to an on (or active) state in which the transistor 430B conducts. Such transition can cause the nMOSFET transistor 430B to switch on, thereby preventing power (such as, flow of electrical current) from being supplied to the light source 410. Instead, electrical current can flow through the nMOSFET transistor 430B toward ground 404. In some cases, a resistor can be positioned between the nMOSFET transistor 430B and ground.

Circuit 400B can operate as follows. The controller 420 can create an electric field at the gate of the nMOSFET transistor 442 causing the transistor to turn on. As a result, power (such as, current or voltage) from the power source 402 can be supplied to the light source 410. Power can also be supplied to the RC network. As power is being supplied to the RC network, capacitor 456 may be charged. Once the capacitor 456 becomes sufficiently charged, electrical energy (such as, voltage) supplied to the gate of the nMOSFET transistor 430B from the capacitor 456 can cause the nMOSFET transistor 430B to turn on. This can prevent power from being supplied to the light source 410, which can cause the light source 410 to turn off (or be deactivated). For example, the capacitor 456 can be charged to about 63.2% of applied direct current (DC) voltage (which can correspond to a time constant of the RC network). Capacitance of the capacitor 456 as well resistance of the resistor 458 can be selected to such that the time constant of the RC network corresponds to a time period associated with safe operation of the light source 410. For example, such time period can be associated with duration of time over which temperature of the light source 410 when the light source operates would not reach or satisfy an unsafe level. Additionally or alternatively, capacitance of the capacitor 456 can be selected to ensure that the capacitor can store sufficient charge to cause the nMOSFET transistor 430B to turn on.

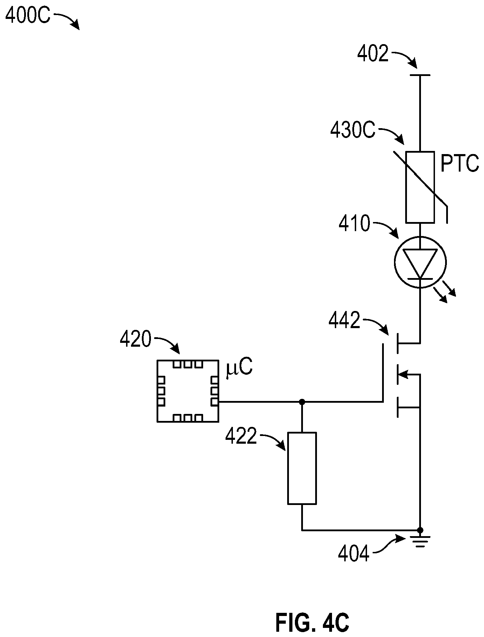

FIG. 4C illustrates a safety circuit 400C. A positive temperature coefficient (PTC) device 430C, such as a PTC resistor (or posistor), PTC thermistor, or the like, can be used in place of switches 430A and 430B of circuits 400A and 400B, respectively. In some cases, a negative temperature coefficient (NTC) device, such as an NTC thermistor, NTC resistor, or the like, can be used in place of or in addition to the PTC device 430C. Resistance of the PTC device 430C can increase with increasing temperature of the light source 410. PTC device 430C can be positioned close to the light source 410. For example, the PTC device 430C can be positioned adjacent to the light source 410. In some instances, the distance between the PTC device 430C and the light source 410 can be 1mm or less or more, 2mm or less or more, 3mm or less or more, 4 mm or less or more, 5mm or less or more, 6mm or less or more, 7mm or less or more, 8mm or less or more, 9mm or less or more, 10mm or less or more, between 11mm and 19mm or less or more, 20mm or less or more, or the like. In operation, the PTC device 430C can limit or reduce the supply of power (such as, flow of electrical current) to the light source 410 as the temperature of the light source increases during operation.

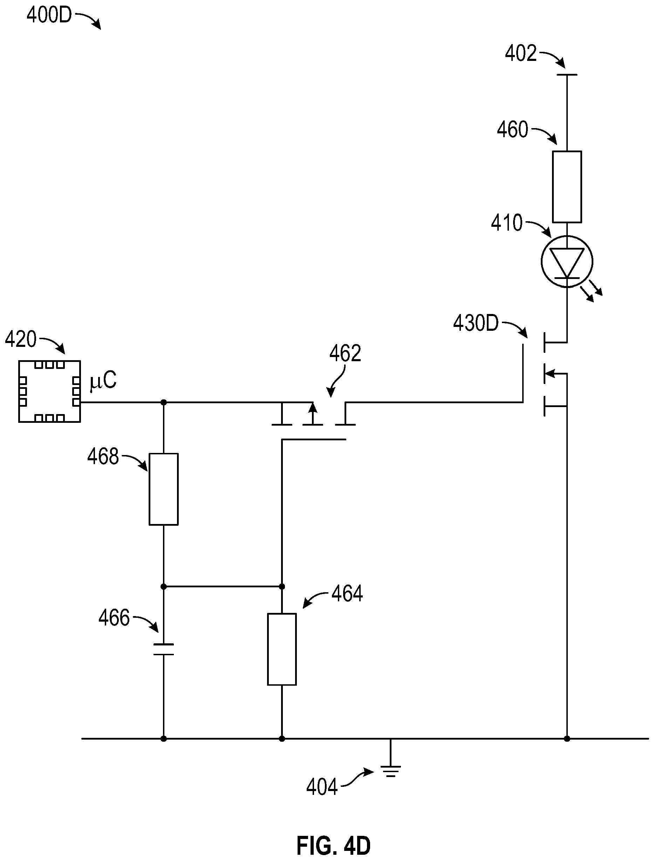

FIG. 4D illustrates a safety circuit 400D. Two switches 462 and 430D can be used in the circuit 400D. The switch 462 can be a pMOSFET transistor. The switch 430D can be an nMOSFET transistor. The switch 462 can be controlled by the controller 420. For example, the controller 420 can be connected to a source (or drain) of the pMOSFET transistor 462 and create an electric field at the source (or drain) of the pMOSFET transistor. Creation of electric field can cause the pMOSFET transistor 462 to transition from an off (or inactive or cutoff) state in which the transistor 462 does not conduct to an on (or active) state in which the transistor 462 conducts. As a result, the switch 462 can transition to the on or active state.

In turn, this can cause the switch 430D to be turned on or become active. For example, the nMOSFET transistor 430D can transition from an off (or inactive or cutoff) state in which the transistor 430D does not conduct to an on (or active) state in which the transistor 430D conducts. Transistor 430D can be controlled in this manner because the gate of the transistor 430D can be connected to an output (for example, drain or source) of the pMOSFET transistor 462. As a result of the switch 430D being in the on state, power can be supplied from the power supply 402 to the light source 410. For example, current can flow to the light source 410 through a resistor 460.

The switch 462 can be controlled by an RC network formed by one or more of a capacitor 466, resistor 466, or resistors 468. The capacitor 466 can be connected to the gate of the pMOSFET transistor 462. Similar to the circuit 400A, once the capacitor 466 is sufficiently charged by power supplied to the RC network by the controller 420, this can cause the switch 462 the switch to turn off. For instance, charging of the capacitor 466 can cause the pMOSFET transistor 462 to transition from the active state to the cutoff state, in which the transistor no longer conducts. Such transition can cause the pMOSFET transistor 462 to transition to the cutoff state, thereby preventing power (such as, voltage) from being supplied to the switch 430D. This can cause the switch 430D to turn off. For example, in response to the gate of the nMOSFET transistor 430D not receiving power (such as, voltage), the nMOSFET transistor 430D can transition from the active state to the cutoff state, in which the transistor no longer conducts. This can result in power no longer being supplied to the light source 410.

As described herein, the capacitor 466 can be charged to about 63.2% of applied direct current (DC) voltage (which can correspond to a time constant of the RC network) in order to cause the switch 462 to turn off. Capacitance of the capacitor 466 as well resistance of one or more of the resistors 464 or 468 can be selected such that the time constant of the RC network corresponds to a time period associated with safe operation of the light source 410. For example, such time period can be associated with duration of time over which temperature of the light source 410 when the light source operates would not reach or satisfy an unsafe level. Additionally or alternatively, capacitance of the capacitor 466 can be selected to ensure that the capacitor can store sufficient charge to cause the pMOSFET transistor 462 to turn off.

In some instances, a safety circuit can utilize an nMOSFET transistor controlled by an RC network. For example, the gate of the nMOSFET transistor can be connected to a capacitor of the RC network. The nMOSFET transistor can be activated in response to the capacitor being sufficiently charged. Activation of the nMOSFET transistor can cause power (such as, current) to be supplied to the light source. Discharging of the capacitor can cause the nMOSFET transistor to be turned off. This can result in power no longer being supplied to the light source. One or more of capacitance of the capacitor or values of one or more resistors of the RC network can be selected such that the time constant of the RC network corresponds to a time period associated with safe operation of the light source and/or that the capacitor can store sufficient charge to cause the nMOSFET transistor to turn on.

In some cases, more than one of any of the switches 430A, 430B, 430C, or 430D or any of the other components illustrated in the circuits 400A to 400D can be used in any of the circuits. In some cases, any of the circuits or portions of the circuits 400A to 400D can be combined.

One or more components illustrated in any of the circuits 400A to 400D can be positioned on the sensor integrated substrate and/or in the control module. For example, the controller 420 can be positioned on the control module. As another example, switches, RC networks, and light sources can be positioned on the sensor integrated substrate. As yet another example, switches and RC networks can be positioned in the control module.

In some cases, a single circuit of any of the circuits 400A to 400D can be used to prevent a single light source (or optical sensor) from overheating. In some cases, a single circuit of the circuits 400A to 400D can be used to prevent more than one light source (or optical sensor) from overheating. In some cases, different light sources (or optical sensors) can be protected from overheating with different circuits 400A to 400D, which may be positioned on a common sensor integrated substrate.

In some instances, resistor-inductor (RL) circuit or network or resistor- capacitor-inductor (RLC) circuit or network can be used in addition to or in place of any of the RC networks described herein.

### Other Variations

Although metal-oxide field-effect transistors (MOSFET) are described in some embodiments, bipolar junction transistors (BJT), field effect transistors (FET), or the like can be alternatively or additionally used. Although RC circuits are described in some embodiments, other circuits configured to generate a time delay can be used alternatively or additionally used. For example, operational amplifier (op-amp) integrator circuits, integrated chip (1C) timer circuits (such as, 555 timer 1C circuits, or the like can be used.

In some cases, one or more electronic components can be positioned on the side of a substrate opposite the side that faces the wound. Systems and methods described herein are equally applicable to such wound contact layers. Although certain embodiments described herein relate to wound dressings, systems and methods disclosed herein are not limited to wound dressings or medical applications. Systems and methods disclosed herein are generally applicable to electronic devices in general, such as electronic devices that can be worn by or applied to a user.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive.

## Claims

1. A wound dressing comprising:
a substantially flexible substrate with a first, wound-facing side supporting a plurality of electronic components and a second side opposite the first side; and
the plurality of electronic components comprising:
an optical sensor comprising a light source;
a resistor-capacitor (RC) network; and
at least one switch electrically connected to the light source and to the RC network, the at least one switch configured to, in a first state, permit power to be supplied to the light source and, in a second state, prevent power being supplied to the light source;
wherein the at least one switch and the RC network are configured to prevent the light source from overheating.

2. The dressing of claim 1, wherein the at least one switch comprises a p-type metal-oxide-semiconductor field-effect transistor (pMOSFET), and wherein the first state comprises an active state of the pMOSFET and the second state comprises a cutoff state of the pMOSFET.

3. The dressing of claim 1, wherein the at least one switch comprises an n-type metal-oxide-semiconductor field-effect transistor (nMOSFET), and wherein the first state comprises a cutoff state of the nMOSFET and the second state comprises an active state of the nMOSFET.

4. The dressing of claim 2 or claim 3, wherein:
the RC network comprises a capacitor; and
i) a gate of the pMOSFET is electrically connected to the capacitor of the RC network, when dependent on claim 2, or
ii) a gate of the nMOSFET is electrically connected to the capacitor of the RC network, when dependent on claim 3.

5. The dressing of claim 4, wherein:
the RC network is electrically connected to a power supply; and
in response to the capacitor of the RC network having being charged,
i) the pMOSFET transitions from the active state to the cutoff state as a result of positive voltage being applied to the gate of the pMOSFET from the capacitor, when dependent on claim 2, or
ii) the nMOSFET transitions from the cutoff state to the active state as a result of positive voltage being applied to the gate of the nMOSFET from the capacitor, when dependent on claim 3.

6. A wound dressing comprising:
a substantially flexible substrate with a first, wound-facing side supporting a plurality of electronic components and a second side opposite the first side; and
the plurality of electronic components comprising:
an optical sensor comprising a light source;
a resistor-capacitor (RC) network; and
at least one switch comprising:
a first switch electrically connected to the RC network; and
a second switch electrically connected to the first switch and to the light source, the second switch configured to, in a first state, permit power to be supplied to the light source and, in a second state, prevent power being supplied to the light source;
wherein the at least one switch and the RC network are configured to prevent the light source from overheating.

7. The dressing of claim 6, wherein:
the first switch comprises a p-type metal-oxide-semiconductor field-effect transistor (pMOSFET) with a gate electrically connected to the RC network; and
the second switch comprises an n-type metal-oxide-semiconductor field-effect transistor (nMOSFET) with a gate electrically connected to an output of the pMOSFET, the first state comprising an active state of the nMOSFET and the second state comprising a cutoff state of the nMOSFET.

8. The dressing of claim 7, wherein:
the RC network comprises a capacitor; and
the gate of the pMOSFET is electrically connected to the capacitor of the RC network,
wherein the pMOSFET is configured to, in an active state, permit power to be supplied to the nMOSFET and, in a cutoff state, prevent power being supplied to the nMOSFET.

9. The dressing of claim 8 wherein:
the RC network is electrically connected to a power supply; and
in response to the capacitor of the RC network having being charged, the pMOSFET transitions from the active state to the cutoff state as a result of positive voltage being applied to the gate of the pMOSFET from the capacitor.

10. The dressing of claim 9, wherein:
in response to the pMOSFET transitioning from the active state to the cutoff state, the nMOSFET transitions from the active state to the cutoff state as a result of voltage no longer being applied to the gate of the nMOSFET.

11. A wound dressing comprising:
a substantially flexible substrate with a first, wound-facing side supporting a plurality of electronic components and a second side opposite the first side; and
the plurality of electronic components comprising:
an optical sensor comprising a light source; and
a positive temperature coefficient (PTC) device electrically connected to the light source, the PTC device configured to limit power supplied to the light source in response to increasing temperature of the light source.

12. The dressing of claim 11, wherein the PTC device is positioned adjacent to the light source.

13. The dressing of any one of the preceding claims, wherein the light source comprises a light emitting diode (LED).

14. The dressing of any one of the preceding claims, further comprising:
a substantially non-stretchable coating applied to at least some of the plurality of electronic components; and
a substantially stretchable coating applied to the first side of the substrate, the stretchable coating applied over the substantially non-stretchable coating.

15. The dressing of claim 14, further comprising a wound contact layer in contact with the substantially stretchable coating, the wound contact layer configured to adhere to a wound.

16. The dressing of claim 14 or claim 15 further comprising:
a wicking layer in contact with the second side of the substrate, the wicking layer configured to facilitate passage of fluid.

## Patentansprüche

1. Ein Wundverband, der Folgendes beinhaltet:
ein im Wesentlichen biegsames Substrat mit einer ersten, der Wunde zugewandten Seite, die eine Vielzahl von elektronischen Komponenten trägt, und einer zweiten Seite gegenüber der ersten Seite; und
wobei die Vielzahl von elektronischen Komponenten Folgendes beinhaltet:
einen optischen Sensor, der eine Lichtquelle beinhaltet;
ein Widerstand-Kondensator-Netzwerk (RC-Netzwerk); und
mindestens einen Schalter, der elektrisch mit der Lichtquelle und dem RC-Netzwerk verbunden ist, wobei der mindestens eine Schalter konfiguriert ist, um in einem ersten Zustand zu erlauben, dass Leistung an die Lichtquelle geliefert wird, und in einem zweiten Zustand zu verhindern, dass Leistung an die Lichtquelle geliefert wird;
wobei der mindestens eine Schalter und das RC-Netzwerk konfiguriert sind, um zu verhindern, dass die Lichtquelle überhitzt.

2. Verband gemäß Anspruch 1, wobei der mindestens eine Schalter einen Metall-Oxid-Halbleiter-Feldeffekttransistor des p-Typs (pMOSFET) beinhaltet, und wobei der erste Zustand einen aktiven Zustand des pMOSFET beinhaltet und der zweite Zustand einen Sperrzustand des pMOSFET beinhaltet.

3. Verband gemäß Anspruch 1, wobei der mindestens eine Schalter einen Metall-Oxid-Halbleiter-Feldeffekttransistor des n-Typs (nMOSFET) beinhaltet, und wobei der erste Zustand einen Sperrzustand des nMOSFET beinhaltet und der zweite Zustand einen aktiven Zustand des nMOSFET beinhaltet.

4. Verband gemäß Anspruch 2 oder Anspruch 3, wobei:
das RC-Netzwerk einen Kondensator beinhaltet; und
i) ein Gate des pMOSFET elektrisch mit dem Kondensator des RC-Netzwerks verbunden ist, wenn abhängig von Anspruch 2, oder
ii) ein Gate des nMOSFET elektrisch mit dem Kondensator des RC-Netzwerks verbunden ist, wenn abhängig von Anspruch 3.

5. Verband gemäß Anspruch 4, wobei:
das RC-Netzwerk elektrisch mit einer Leistungsversorgung verbunden ist; und
als Reaktion darauf, dass der Kondensator des RC-Netzwerks geladen worden ist,
i) der pMOSFET von dem aktiven Zustand in den Sperrzustand übergeht, als ein Resultat davon, dass positive Spannung von dem Kondensator an das Gate des pMOSFET angelegt wird, wenn abhängig von Anspruch 2, oder
ii) der nMOSFET von dem Sperrzustand in den aktiven Zustand übergeht, als ein Resultat davon, dass positive Spannung von dem Kondensator an das Gate des nMOSFET angelegt wird, wenn abhängig von Anspruch 3.

6. Ein Wundverband, der Folgendes beinhaltet:
ein im Wesentlichen biegsames Substrat mit einer ersten, der Wunde zugewandten Seite, die eine Vielzahl von elektronischen Komponenten trägt, und einer zweiten Seite gegenüber der ersten Seite; und
wobei die Vielzahl von elektronischen Komponenten Folgendes beinhaltet:
einen optischen Sensor, der eine Lichtquelle beinhaltet;
ein Widerstand-Kondensator-Netzwerk (RC-Netzwerk); und
mindestens einen Schalter, der Folgendes beinhaltet:
einen ersten Schalter, der elektrisch mit dem RC-Netzwerk verbunden ist; und
einen zweiten Schalter, der elektrisch mit dem ersten Schalter und mit der Lichtquelle verbunden ist, wobei der zweite Schalter konfiguriert ist, um in einem ersten Zustand zu erlauben, dass Leistung an die Lichtquelle geliefert wird, und in einem zweiten Zustand zu verhindern, dass Leistung an die Lichtquelle geliefert wird;
wobei der mindestens eine Schalter und das RC-Netzwerk konfiguriert sind, um zu verhindern, dass die Lichtquelle überhitzt.

7. Verband gemäß Anspruch 6, wobei:
der erste Schalter einen Metall-Oxid-Halbleiter-Feldeffekttransistor des p-Typs (pMOSFET) mit einem Gate, das elektrisch mit dem RC-Netzwerk verbunden ist, beinhaltet; und
wobei der zweite Schalter einen Metall-Oxid-Halbleiter-Feldeffekttransistor des n-Typs (nMOSFET) mit einem Gate, das elektrisch mit einem Ausgang des pMOSFET verbunden ist, beinhaltet, wobei der erste Zustand einen aktiven Zustand des nMOSFET beinhaltet und der zweite Zustand einen Sperrzustand des nMOSFET beinhaltet.

8. Verband gemäß Anspruch 7, wobei:
das RC-Netzwerk einen Kondensator beinhaltet; und
das Gate des pMOSFET elektrisch mit dem Kondensator des RC-Netzwerks verbunden ist,
wobei der pMOSFET konfiguriert ist, um in einem aktiven Zustand zu erlauben, dass Leistung an den nMOSFET geliefert wird, und in einem Sperrzustand zu verhindern, dass Leistung an den nMOSFET geliefert wird.

9. Verband gemäß Anspruch 8, wobei:
das RC-Netzwerk elektrisch mit einer Leistungsversorgung verbunden ist; und
als Reaktion darauf, dass der Kondensator des RC-Netzwerks geladen worden ist,
der pMOSFET von dem aktiven Zustand in den Sperrzustand übergeht, als ein Resultat davon, dass positive Spannung von dem Kondensator an das Gate des pMOSFET angelegt wird.

10. Verband gemäß Anspruch 9, wobei:
als Reaktion darauf, dass der pMOSFET von dem aktiven Zustand in den Sperrzustand übergeht, der nMOSFET von dem aktiven Zustand in den Sperrzustand übergeht, als ein Resultat davon, dass Spannung nicht länger an das Gate des nMOSFET angelegt wird.

11. Ein Wundverband, der Folgendes beinhaltet:
ein im Wesentlichen biegsames Substrat mit einer ersten, der Wunde zugewandten Seite, die eine Vielzahl von elektronischen Komponenten trägt, und einer zweiten Seite gegenüber der ersten Seite; und
wobei die Vielzahl von elektronischen Komponenten Folgendes beinhaltet:
einen optischen Sensor, der eine Lichtquelle beinhaltet; und
eine Vorrichtung mit positivem Temperaturkoeffizienten (PTC), die elektrisch mit der Lichtquelle verbunden ist, wobei die PTC-Vorrichtung konfiguriert ist, um Leistung, die an die Lichtquelle geliefert wird, als Reaktion auf das Zunehmen der Temperatur der Lichtquelle zu begrenzen.

12. Verband gemäß Anspruch 11, wobei die PTC-Vorrichtung neben der Lichtquelle positioniert ist.

13. Verband gemäß einem der vorhergehenden Ansprüche, wobei die Lichtquelle eine lichtemittierende Diode (LED) beinhaltet.

14. Verband gemäß einem der vorhergehenden Ansprüche, der ferner Folgendes beinhaltet:
eine im Wesentlichen nicht dehnbare Beschichtung, die auf mindestens einige der Vielzahl von elektronischen Komponenten aufgebracht ist; und
eine im Wesentlichen dehnbare Beschichtung, die auf die erste Seite des Substrats aufgebracht ist, wobei die dehnbare Beschichtung über der im Wesentlichen nicht dehnbaren Beschichtung aufgebracht ist.

15. Verband gemäß Anspruch 14, der ferner eine Wundkontaktschicht in Kontakt mit der im Wesentlichen dehnbaren Beschichtung beinhaltet, wobei die Wundkontaktschicht konfiguriert ist, um an einer Wunde zu haften.

16. Verband gemäß Anspruch 14 oder Anspruch 15, der ferner Folgendes beinhaltet:
eine Dochtschicht in Kontakt mit der zweiten Seite des Substrats, wobei die Dochtschicht konfiguriert ist, um den Durchgang von Fluid zu erleichtern.

## Revendications

1. Un pansement pour plaie comprenant :
un substrat substantiellement souple avec un premier côté tourné vers la plaie supportant une pluralité de composants électroniques et un deuxième côté à l'opposé du premier côté ; et
la pluralité de composants électroniques comprenant :
un capteur optique comprenant une source lumineuse ;
un réseau résistance-capacité (RC) ; et
au moins un interrupteur raccordé électriquement à la source lumineuse et au réseau RC, l'au moins un interrupteur étant configuré pour, dans un premier état, permettre que la source lumineuse soit alimentée en puissance et, dans un deuxième état, empêcher que la source lumineuse ne soit alimentée en puissance ;
dans lequel l'au moins un interrupteur et le réseau RC sont configurés pour empêcher la source lumineuse de surchauffer.

2. Le pansement de la revendication 1, dans lequel l'au moins un interrupteur comprend un transistor à effet de champ métal-oxyde semiconducteur de type p (pMOSFET), et dans lequel le premier état comprend un état actif du pMOSFET et le deuxième état comprend un état éteint du pMOSFET.

3. Le pansement de la revendication 1, dans lequel l'au moins un interrupteur comprend un transistor à effet de champ métal-oxyde semiconducteur de type n (nMOSFET), et dans lequel le premier état comprend un état éteint du nMOSFET et le deuxième état comprend un état actif du nMOSFET.

4. Le pansement de la revendication 2 ou de la revendication 3, dans lequel :
le réseau RC comprend un condensateur ; et
i) une grille du pMOSFET est raccordée électriquement au condensateur du réseau RC, lorsque dépendant de la revendication 2, ou
ii) une grille du nMOSFET est raccordée électriquement au condensateur du réseau RC, lorsque dépendant de la revendication 3.

5. Le pansement de la revendication 4, dans lequel :
le réseau RC est raccordé électriquement à une alimentation en puissance ; et
en réponse au fait que le condensateur du réseau RC a été chargé,
i) le pMOSFET passe de l'état actif à l'état éteint en conséquence du fait qu'une tension positive est appliquée à la grille du pMOSFET en provenance du condensateur, lorsque dépendant de la revendication 2, ou
ii) le nMOSFET passe de l'état éteint à l'état actif en conséquence du fait qu'une tension positive est appliquée à la grille du nMOSFET en provenance du condensateur, lorsque dépendant de la revendication 3.

6. Un pansement pour plaie comprenant :
un substrat substantiellement souple avec un premier côté tourné vers la plaie supportant une pluralité de composants électroniques et un deuxième côté à l'opposé du premier côté ; et
la pluralité de composants électroniques comprenant :
un capteur optique comprenant un source lumineuse ;
un réseau résistance-capacité (RC) ; et
au moins un interrupteur comprenant :
un premier interrupteur raccordé électriquement au réseau RC ; et
un deuxième interrupteur raccordé électriquement au premier interrupteur et à la source lumineuse, le deuxième interrupteur étant configuré pour,
dans un premier état, permettre que la source lumineuse soit alimentée en puissance et, dans un deuxième état, empêcher que la source lumineuse ne soit alimentée en puissance ;
dans lequel l'au moins un interrupteur et le réseau RC sont configurés pour empêcher la source lumineuse de surchauffer.

7. Le pansement de la revendication 6, dans lequel :
le premier interrupteur comprend un transistor à effet de champ métal-oxyde semiconducteur de type p (pMOSFET) avec une grille raccordée électriquement au réseau RC ; et
le deuxième interrupteur comprend un transistor à effet de champ métal-oxyde semiconducteur de type n (nMOSFET) avec une grille raccordée électriquement à une sortie du pMOSFET, le premier état comprenant un état actif du nMOSFET et le deuxième état comprenant un état éteint du nMOSFET.

8. Le pansement de la revendication 7, dans lequel :
le réseau RC comprend un condensateur ; et
la grille du pMOSFET est raccordée électriquement au condensateur du réseau RC, dans lequel le pMOSFET est configuré pour, dans un état actif, permettre que le nMOSFET soit alimenté en puissance et, dans un état éteint, empêcher que le nMOSFET ne soit alimenté en puissance.

9. Le pansement de la revendication 8, dans lequel :
le réseau RC est raccordé électriquement à une alimentation en puissance ; et
en réponse au fait que le condensateur du réseau RC a été chargé, le pMOSFET passe de l'état actif à l'état éteint en conséquence du fait qu'une tension positive est appliquée à la grille du pMOSFET en provenance du condensateur.

10. Le pansement de la revendication 9, dans lequel :
en réponse au fait que le pMOSFET passe de l'état actif à l'état éteint, le nMOSFET passe de l'état actif à l'état éteint en conséquence du fait qu'une tension n'est plus appliquée à la grille du nMOSFET.

11. Un pansement pour plaie comprenant :
un substrat substantiellement souple avec un premier côté tourné vers la plaie supportant une pluralité de composants électroniques et un deuxième côté à l'opposé du premier côté ; et
la pluralité de composants électroniques comprenant :
un capteur optique comprenant une source lumineuse ; et
un dispositif à coefficient de température positif (CTP) raccordé électriquement à la source lumineuse, le dispositif CTP étant configuré pour limiter une puissance alimentant la source lumineuse en réponse au fait que la température de la source lumineuse augmente.

12. Le pansement de la revendication 11, dans lequel le dispositif CTP est positionné de façon adjacente à la source lumineuse.

13. Le pansement de l'une quelconque des revendications précédentes, dans lequel la source lumineuse comprend une diode électroluminescente (DEL).

14. Le pansement de l'une quelconque des revendications précédentes, comprenant en outre :
un revêtement substantiellement non étirable appliqué sur au moins certains des composants de la pluralité de composants électroniques ; et
un revêtement substantiellement étirable appliqué sur le premier côté du substrat, le revêtement étirable étant appliqué par-dessus le revêtement substantiellement non étirable.

15. Le pansement de la revendication 14, comprenant en outre une couche de contact avec la plaie en contact avec le revêtement substantiellement étirable, la couche de contact avec la plaie étant configurée pour adhérer à une blessure.

16. Le procédé de la revendication 14 ou de la revendication 15 comprenant en outre :
une couche à effet de mèche en contact avec le deuxième côté du substrat, la couche à effet de mèche étant configurée pour faciliter le passage de fluide.
